# EUROPÄISCHE PATENTSCHRIFT

(11) **EP 0 777 639 B1**
(45) Veröffentlichungstag und Bekanntmachung des Hinweises auf die Patenterteilung: **09.12.1998**
(21) Anmeldenummer: 95929832.4
(22) Anmeldetag: 10.08.1995
(51) Int. Cl.: C07C 45/41

(54) **VERFAHREN ZUR HERSTELLUNG VON AROMATISCHEN ALDEHYDEN**
PROCESS FOR PREPARING AROMATIC ALDEHYDES
PROCEDE DE PREPARATION D'ALDEHYDES AROMATIQUES

(30) Priorität: 16.08.1994 DE 4428994; 22.12.1994 DE 4446009
(43) Veröffentlichungstag der Anmeldung: 11.06.1997
(73) Patentinhaber: BASF AKTIENGESELLSCHAFT, 67056 Ludwigshafen (DE)
(72) Erfinder: FISCHER, Rolf, D-69121 Heidelberg (DE); IRGANG, Matthias, D-69121 Heidelberg (DE); SCHNURR, Werner, D-67273 Herxheim (DE); WULFF-DÖRING, Joachim, D-67227 Frankenthal (DE)
(86) Internationale Anmeldenummer: EP9503167
(87) Internationale Veröffentlichungsnummer: WO9605161

(56) Entgegenhaltungen:
- EP-A- 0 150 961
- US-A- 4 328 373

## Beschreibung

Die vorliegende Erfindung betrifft ein Verfahren zur Herstellung von aromatischen Aldehyden durch Umsetzung entsprechender Carbonsäuren oder deren Ester mit Wasserstoff in der Gasphase in Gegenwart von Zirkonoxid und Elementen der Lanthaniden enthaltenden Katalysatoren.

Es ist bekannt, Carbonsäuren wie Benzoesäure oder Cyclohexancarbonsäure oder ihre Ester durch Hydrierung in der Gasphase in die entsprechenden Aldehyde zu überführen.

Aus der US-A-3,935,265 ist bekannt, daß man Alkylester aromatischer Carbonsäuren bei 400 bis 600°C an Al₂O₃ mit Wasserstoff hydrieren kann. Beispielsweise wird Benzoesäuremethylester mit einer Selektivität von 37 % (Umsatz: 39 %) zu Benzaldehyd umgesetzt. Weiterhin werden z.B. Ru/Sn- (EP-A-539 274), Manganoxid-(EP-A-290 096, US-A-4,585,899), Eisenoxid- (EP-A-304 853), Vanadiumoxid und/oder Titandioxid- (US-A-4,950,799, EP-A-414 065), Cu/Y₂O₃- (US-A-4,585,900), Cr₂O₃/ZrO₂- und Ce/ ZrO₂-Katalysatoren mit geringer BET-Oberfläche (EP-A-150 961), oder Lanthanidoxide/Al₂O₃-Katalysatoren (US-A-4,328,373, EP-A-101 111) für die Hydrierung von aromatischen Carbonsäuren eingesetzt.

Bei den bekannten Hydrierverfahren werden in den meisten Fällen, zum Teil bedingt durch sehr hohe Hydriertemperaturen, nur unbefriedigende Ausbeuten und Selektivitäten erzielt.

Der vorliegenden Erfindung lag daher die Aufgabe zugrunde, den zuvor genannten Nachteilen abzuhelfen.

Demgemäß wurde ein neues und verbessertes Verfahren zur Herstellung von aromatischen Aldehyden der allgemeinen Formel I 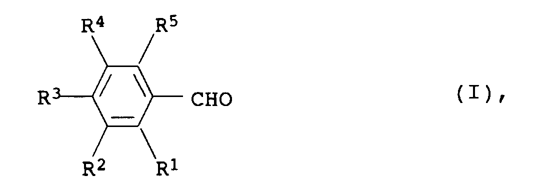 in der
- R¹,R²,R³,R⁴ und R⁵: Wasserstoff, C₁- bis C₆-Alkyl, C₃- bis C₈-Cycloalkyl, Aryl, Hydroxy, C₁- bis C₄-Alkoxy, Phenoxy, C₇- bis C₁₂-Alkylphenyl, C₇- bis C₁₂-Phenylalkyl, Amino, Methylamino, Dimethylamino oder Halogen und
- R¹: zusätzlich Formyl oder COOR⁶
- R⁶: Wasserstoff, C₁- bis C₆-Alkyl, C₃- bis C₈-Cycloalkyl, Aryl, C₇- bis C₁₂-Alkylphenyl oder C₇- bis C₁₂-Phenylalkyl,
bedeuten, gefunden, welches dadurch gekennzeichnet ist, daß man aromatische Carbonsäuren oder deren Ester der allgemeinen Formel II 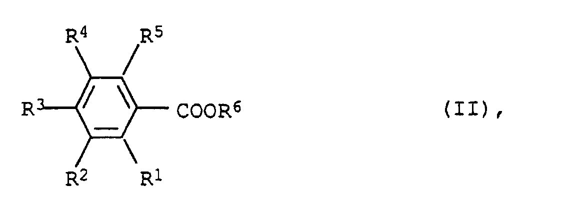 in der
- R¹,R²,R³,R⁴,R⁵ und R⁶: die obengenannte Bedeutung haben oder in der
- R²,R³,R⁴ und R⁵: die obengenannte Bedeutung haben und
- R⁶ und R¹: gemeinsam eine -CH₂-Brücke bilden,
mit Wasserstoff in der Gasphase bei Temperaturen von 200 bis 450°C und Drücken von 0,1 bis 20 bar in Gegenwart eines Katalysators, dessen BET-Oberfläche 20 bis 150 m²/g beträgt und dessen katalytisch aktive Masse 80 bis 99,9 Gew.-% Zirkonoxid und 0,1 bis 20 Gew.-% eines oder mehrere Elemente der Lanthaniden enthält, umsetzt.

Das erfindungsgemäße Verfahren läßt sich wie folgt durchführen:

Die erfindungsgemäße Hydrierung der aromatischen Carbonsäuren oder deren Ester II mit Wasserstoff in Gegenwart eines Katalysators, dessen katalytisch aktive Masse 60 bis 99,9, insbesondere 80 bis 99,9 Gew.-% Zirkonoxid und 0,1 bis 40, insbesondere 0,1 bis 20 Gew.-% eines oder mehrere Elemente der Lanthaniden enthält, wird in der Regel bei Temperaturen von 200 bis 450°C, bevorzugt 250 bis 400°C, besonders bevorzugt 300 bis 380°C und Drücken von 0,1 bis 20 bar, bevorzugt 0,7 bis 5 bar, besonders bevorzugt Atmosphärendruck (Normaldruck) durchgeführt. Die erforderliche Temperatur und der erforderliche Druck sind abhängig von der Katalysatoraktivität und der thermischen Stabilität von Edukt und Produkt.

Als Katalysatoren eignen sich Trägerkatalysatoren, bevorzugt Vollkatalysatoren von Zirkonoxid in kubischer, tetragonaler oder monokliner Phase, bevorzugt in monokliner Phase, die bevorzugt mit einem oder mehreren Elementen aus der Lanthaniden-Reihe dotiert sind. Die katalytisch aktive Masse enthält in der Regel 80 bis 99,9 Gew.-%, bevorzugt 90 bis 99,9 Gew.-%, besonders bevorzugt 92 bis 99 Gew.-% Zirkonoxid und 0,1 bis 20 Gew.-% eines oder mehrere Elemente der Lanthaniden, bevorzugt 0,1 bis 10 Gew.-% Lanthan, Cer, Praseodym, Neodym, Samarium, Europium oder deren Gemische, besonders bevorzugt 1 bis 8 Gew.-% Lanthan-(III)-oxid. Die Dotierung erfolgt in der Regel durch Tränken des Zirkonoxids mit Salzlösungen (wäßrig oder alkoholisch) der Lanthaniden.

Der Katalysator kann zusätzlich weitere Dotierungen (z.B. Chrom, Eisen, Yttrium, Mangan) in Mengen von 0,001 bis 10 Gew.-% enthalten. Bevorzugt sind Katalysatoren ohne solche Zusätze.

Die BET-Oberfläche des Zirkonoxids beträgt 20 bis 150 m²/g, besonders bevorzugt 40 bis 120 m²/g.

Derartige Katalysatoren werden in bekannter Weise z. B. durch Tränken vorgeformter Träger wie Pellets, Kugeln oder Stränge, Trocknen und Calcinieren hergestellt.

Die bevorzugt verwendeten Trägerkatalysatoren zeigen über einen längeren Zeitraum hohe Aktivität. Desaktivierte Katalysatoren lassen sich durch Behandlung mit molekularen Sauerstoff enthaltenden Gasen, z.B. Luft, bei Temperaturen von 350 bis 500°C regenerieren.

Im allgemeinen hält man eine Katalysatorbelastung von 0,01 bis 10, vorzugsweise 0,01 bis 3 kg Carbonsäure(ester) je kg Katalysator und Stunde ein.

Die Wasserstoffkonzentration im Eingangsgas richtet sich nach der Carbonsäure(ester)konzentration. Das Molverhältnis von Wasserstoff zu Carbonsäure(ester) beträgt in der Regel 2:1 bis 100:1, bevorzugt 10:1 bis 70:1. Als Wasserstoffquelle kann auch Ameisensäure eingesetzt werden.

Vorteilhaft kann auch der Zusatz eines inerten Verdünnungsmittels sein. In der Regel werden Stickstoff, Wasser oder gasförmige, unter den Reaktionsbedingungen inerte Verbindungen wie z.B. Kohlenwasserstoffe, Aromaten oder Ether verwendet.

Die Umsetzung kann in der Gasphase, kontinuierlich als Festbettreaktion mit fest angeordnetem Katalysator, beispielsweise in Sumpf- oder Rieselfahrweise oder als Wirbelbettreaktion mit in auf- und abwirbelnder Bewegung befindlichem Katalysator durchgeführt werden. Bevorzugt ist das Arbeiten im Festbett.

Zur Steigerung der Selektivität können bei der Hydrierung gebildete Nebenprodukte, z.B. Alkohole, in die Synthese zurückgeführt werden.

Die Substituenten R¹, R², R³, R⁴, R⁵ und R⁶ in den Verbindungen I und II haben unabhängig voneinander die folgenden Bedeutungen:
R¹,R²,R³,R⁴,R⁵ und R⁶
- Wasserstoff,
- C₁- bis C₆-Alkyl, bevorzugt C₁- bis C₄-Alkyl wie Methyl, Ethyl, n-Propyl, iso-Propyl, n-Butyl, iso-Butyl, sec.-Butyl und tert.-Butyl, besonders bevorzugt Methyl und Ethyl,
- C₃- bis C₈-Cycloalkyl wie Cyclopropyl, Cyclobutyl, Cyclopentyl, Cyclohexyl, Cycloheptyl und Cyclooctyl, bevorzugt Cyclopentyl, Cyclohexyl und Cyclooctyl, besonders bevorzugt Cyclopentyl und Cyclohexyl,
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- C₇- bis C₁₂-Alkylphenyl wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl, bevorzugt 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl und 5 3,5-Dimethylphenyl, besonders bevorzugt 2-Methylphenyl, 3-Methylphenyl, 4-Methylphenyl,
- C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenylbutyl, 2-Phenyl-butyl, 3-Phenyl-butyl und 4-Phenyl-butyl, bevorzugt Benzyl, 1-Phenethyl und 2-Phenethyl, besonders bevorzugt Benzyl,
R²,R³ und R⁴
- Hydroxy,
- C₁- bis C₄-Alkoxy wie Methoxy, Ethoxy, n-Propoxy, iso-Propoxy, n-Butoxy, iso-Butoxy, sec.-Butoxy und tert.-Butoxy, bevorzugt Methoxy, Ethoxy, n-Propoxy und iso-Propoxy, besonders bevorzugt Methoxy und Ethoxy,
- Phenoxy,
- Amino,
- Methylamino,
- Dimethylamino,
- Halogen wie Fluor, Chlor, Brom und Jod, bevorzugt Fluor, Chlor und Brom, besonders bevorzugt Chlor und Brom und
R², R³ oder R⁴
- Formyl (-CHO) oder COOR⁶,

R⁶ und R¹ können weiterhin in Formel II gemeinsam eine -CH₂-Brücke bilden.

Das Substitutionsmuster ist beliebig, d.h. die Substituenten können z.B. sowohl in 2,3,4-, 2,3,5-, 2,3,6-, 3,4,5-, 3,4,6- als auch in 4,5,6-Stellung angeordnet sein.

Als Einsatzstoffe dienen aromatische Mono- oder Dicarbonsäuren bzw. Carbonsäureester II, z.B. Benzoesäure(ester), Terephthalsäure(ester), Phthalsäure(ester), i-Phthalsäure(ester), alkylsubstituierte, alkoxysubstituierte, dialkylsubstituierte oder alkoxyalkylsubstituierte Carbonsäuren(ester). Als Ester werden bevorzugt Methyl-, Ethyl-, Propyl-, iso-Propyl-, Butyl-, iso-Butyl-, tert.-Butyl-, Cyclopentyl-, Cyclohexyl-, Phenyl- und Benzyl-Ester eingesetzt. Besonders bevorzugt sind Benzoesäure, Terephthalsäure, Phthalsäure, 2-, 3- und 4-Methylbenzoesäure, 4-iso-Propylbenzoesäure, 4-tert.-Butylbenzoesäure, 4-Methoxybenzoesäure, Phthalid, Vanillinsäure und die jeweiligen Methylester.

Bei der Hydrierung von Dicarbonsäuren(estern) entstehen zunächst Aldehydcarbonsäuren(ester), die gegebenenfalls auch als Zwischenprodukte Verwendung finden. Die maximale Aldehydcarbonsäure-(ester)-Ausbeute wird bei unvollständigem Umsatz erhalten.

Bei Umsatzsteigerung wird durch Weiterhydrierung bevorzugt Dialdehydbildung beobachtet.

Mit Hilfe des erfindungsgemäßen Verfahrens ist es möglich, bisher schwer zugängliche Aldehyde auf einfache Weise selektiv herzustellen.

Die Aldehyde I eignen sich als Geruchs- und Geschmacksstoffe bzw. als Zwischenprodukte z.B. für Pharma- und Pflanzenschutzwirkstoffe (Ullmann's Encyclopedia of Industrial Chemistry, Vol. A3, S. 469-74) .

### Beispiele

### Katalysatorherstellung

### Beispiel 1

Monoklines Zirkondioxid (BET-Oberfläche: 40 bis 85 m²/g) in Form von Tabletten (Katalysator A, E, F) oder Strängen (Katalysator B, C, D) wurde mit einer wäßrigen Lösung des Lanthanid-Element-Nitrats (bzw. der Lanthanid-Element-Nitrate) unter guter Durchmischung getränkt und 2 h bei Raumtemperatur gehalten. Anschließend wurde der Katalysator 15 Stunden bei 120°C getrocknet und anschließend 2 bis 4 Stunden bei 400 bis 500°C getempert.

Die so hergestellten Katalysatoren hatten folgenden Lanthanid-Gehalt:
Katalysator A (Oberfläche 67 m²/g): 3 Gew.-% Lanthan
Katalysator B (Oberfläche 46 m²/g): 3 Gew.-% Praseodym
Katalysator C (Oberfläche 46 m²/g): 3 Gew.-% Cer
Katalysator D (Oberfläche 46 m²/g): 3 Gew.-% Lanthanide (Verteilung: 48 % CeO₂, 26,3 % La₂O₃, 5,7 % Pr₂O₃ und 19,7 % Nd₂O₃).
Katalysator E (Oberfläche 85 m²/g): 3 Gew.-% Lanthan
Katalysator F (Oberfläche 53 m²/g): 3 Gew.-% Lanthan

### Beispiel 2

Pro Stunde wurden 4 bis 8 g aromatische Carbonsäure(ester) II mit oder ohne Lösungsmittel (THF) in einen Verdampfer (< 300°C) geleitet und von dort mit 100 l/h Wasserstoff über 100 g Katalysator in Rieselfahrweise geleitet. Der gasförmige Reaktionsaustrag wurde in Kühlfallen kondensiert und gaschromatographisch analysiert. Die Ergebnisse sind in Tabelle 1 zusammengefaßt.

### Beispiel 3

Pro Stunde wurden 6 g Terephthalsäuredimethylester (als Schmelze) mit 100 l/h Wasserstoff verdampft und bei 350°C über 100 g Katalysator E in Rieselfahrweise geleitet. Der gasförmige Reaktionsaustrag wurde in Kühlfallen kondensiert und gaschromatographisch analysiert. Als Hauptprodukt erhält man 32 % Terephthaldialdehyd und 27 % 4-Formylbenzoesäuremethylester (Umsatz: 78 %).

### Beispiel 4

Pro Stunde wurden 8 g 2-Methylbenzoesäure (als Schmelze) mit 200 l/h Wasserstoff verdampft und bei 350°C über 100 g Katalysator F in Rieselfahrweise geleitet. Der gasförmige Reaktionsaustrag wurde in Kühlfallen kondensiert und gaschromatographisch analysiert. Die 2-Methylbenzaldehyd-Ausbeute betrug 93 % (Umsatz 99 %).

### Beispiel 5

Pro Stunde wurden 5 g 3-Methylbenzoesäure (als Schmelze) mit 100 l/h Wasserstoff verdampft und bei 360°C über 100 g Katalysator F in Rieselfahrweise geleitet. Der gasförmige Reaktionsaustrag wurde in Kühlfallen kondensiert und gaschromatographisch analysiert. Die 3-Methylbenzaldehyd-Ausbeute betrug 92 % (Umsatz 99 %).

### Beispiel 6

Pro Stunde wurden 8 g Phthalid (als Schmelze) mit 100 l/h Wasserstoff verdampft und bei 400°C über 100 g Katalysator F in Rieselfahrweise geleitet. Der gasförmige Reaktionsaustrag wurde in Kühlfallen kondensiert und gaschromatographisch analysiert. Die 2-Methylbenzaldehyd-Ausbeute betrug 16 % (Umsatz 26 %).

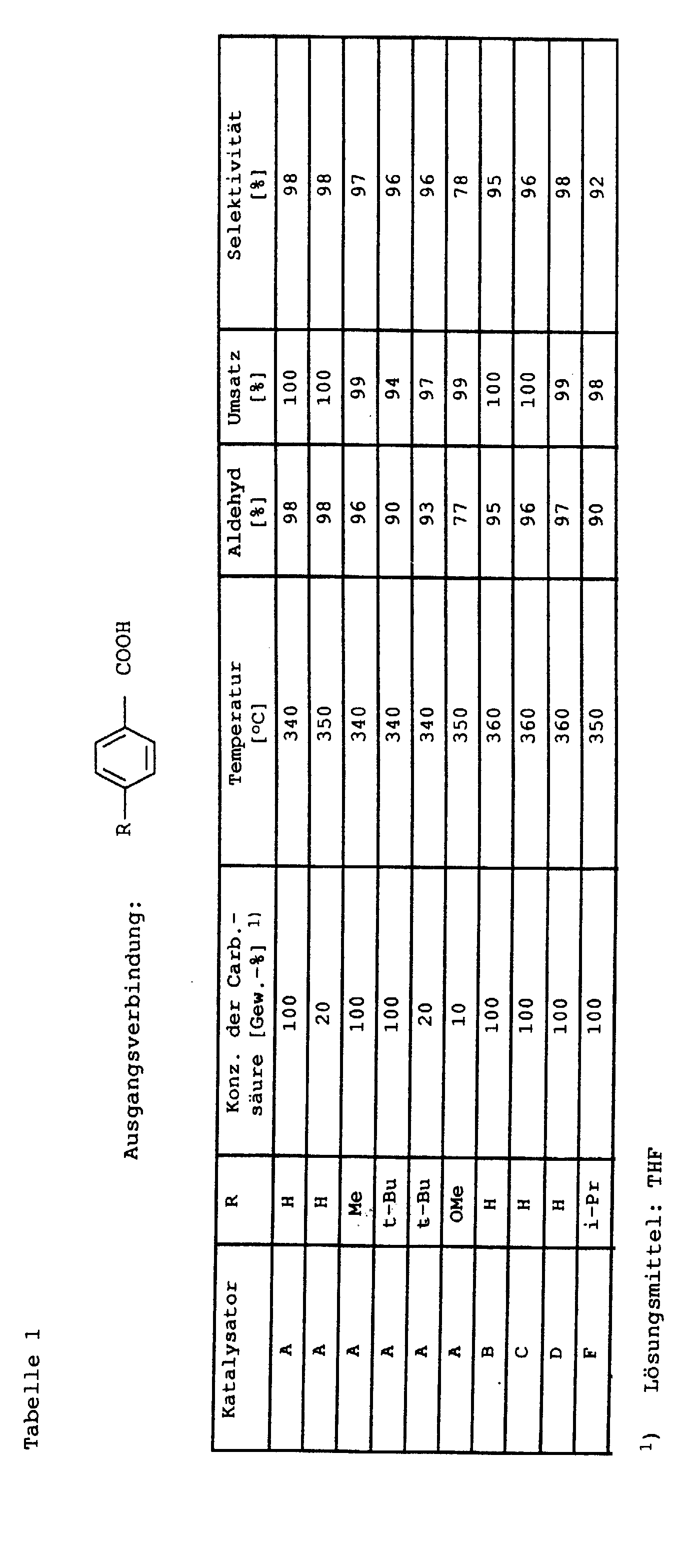

## Patentansprüche

1. Verfahren zur Herstellung von aromatischen Aldehyden der allgemeinen Formel I 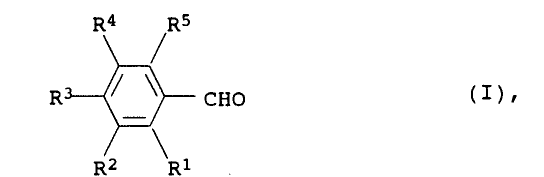 in der
R¹,R²,R³,R⁴ und R⁵ Wasserstoff, C₁- bis C₆-Alkyl, C₃- bis C₈-Cycloalkyl, Aryl, Hydroxy, C₁-bis C₄-Alkoxy, Phenoxy, C₇- bis C₁₂-Alkylphenyl, C₇- bis C₁₂-Phenylalkyl, Amino, Methylamino, Dimethylamino oder Halogen und
R², R³ oder R⁴ zusätzlich Formyl oder COOR⁶
R⁶ Wasserstoff, C₁- bis C₆-Alkyl, C₃- bis C₈-Cycloalkyl, Aryl, C₇- bis C₁₂-Alkylphenyl oder C₇- bis C₁₂-Phenylalkyl,
bedeuten, dadurch gekennzeichnet, daß man aromatische Carbonsäuren oder deren Ester der allgemeinen Formel II 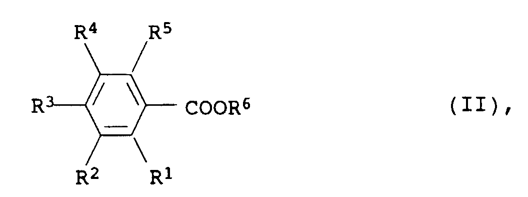 in der
R¹,R²,R³,R⁴,R⁵ und R⁶ die obengenannte Bedeutung haben oder in der
R²,R³,R⁴ und R⁵ die obengenannte Bedeutung haben und
R⁶ und R¹ gemeinsam eine -CH₂-Brücke bilden,
mit Wasserstoff in der Gasphase bei Temperaturen von 200 bis 450°C und Drücken von 0,1 bis 20 bar in Gegenwart eines Katalysators, dessen BET-Oberfläche 20 bis 150 m²/g beträgt und dessen katalytisch aktive Masse 80 bis 99,9 Gew.-% Zirkonoxid und 0,1 bis 20 Gew.-% eines oder mehrere Elemente der Lanthaniden enthält, umsetzt.

2. Verfahren zur Herstellung von aromatischen Aldehyden nach Anspruch 1, dadurch gekennzeichnet, daß der Katalysator als katalytisch aktive Masse 90 bis 99,9 Gew.-% Zirkonoxid und als Element der Lanthaniden 0,1 bis 10 Gew.-% Lanthan, Cer, Praseodym, Neodym, Samarium, Europium oder deren Gemische enthält.

3. Verfahren zur Herstellung von aromatischen Aldehyden nach Anspruch 1, dadurch gekennzeichnet, daß der Katalysator als katalytisch aktive Masse 92 bis 99 Gew.-% Zirkonoxid und 1 bis 8 Gew.-% Lanthan-(III)-oxid enthält.

4. Verfahren zur Herstellung von aromatischen Aldehyden nach Anspruch 1, dadurch gekennzeichnet, daßdas Zirkondioxid monoklin ist.

5. Verfahren zur Herstellung von aromatischen Aldehyden nach Anspruch 1, dadurch gekennzeichnet, daß man Benzoesäure, 2-Methylbenzoesäure, 3-Methylbenzoesäure, 4-Methylbenzoesäure, 4-iso-Propylbenzoesäure, 4-Methoxybenzoesäure, Phthalid oder Terephthalsäure oder ihre Ester zum entsprechenden Mono- oder Dialdehyd hydriert.

6. Verfahren zur Herstellung von aromatischen Aldehyden nach Anspruch 1, dadurch gekennzeichnet, daß das Molverhältnis von Wasserstoff zu Carbonsäure(ester) 2:1 bis 100:1 beträgt.

7. Verfahren zur Herstellung von aromatischen Aldehyden nach Anspruch 1, dadurch gekennzeichnet, daß man die Umsetzung im Festbett durchführt.

## Claims

1. A process for preparing aromatic aldehydes of the general formula I 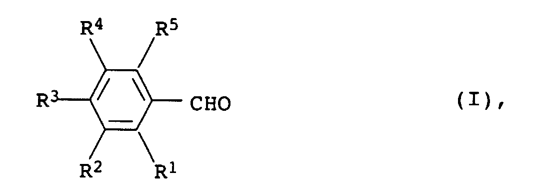 where
R¹, R², R³, R⁴ and R⁵ are hydrogen, C₁- to C₆-alkyl, C₃-to C₈-cycloalkyl, aryl, hydroxyl, C₁- to C₄-alkoxy, phenoxy, C₇- to C₁₂-alkylphenyl, C₇- to C₁₂-phenyl-alkyl, amino, methylamino, dimethylamino or halogen and
R², R³ or R⁴ is additionally formyl or COOR⁶
R⁶ is hydrogen, C₁- to C₆-alkyl, C₃-to C₈-cycloalkyl, aryl, C₇- to C₁₂-alkylphenyl or C₇- to C₁₂-phenylalkyl,
which comprises reacting aromatic carboxylic acids or their esters of the general formula II 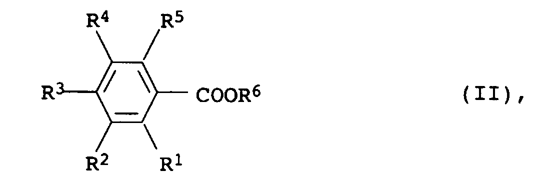 where
R¹, R², R³, R⁴, R⁵ and R⁶ have the abovementioned meanings or where
R², R³, R⁴ and R⁵ have the abovementioned meanings and
R⁶ and R¹ together form a -CH₂- bridge,
with hydrogen in the gas phase at from 200 to 450°C and from 0.1 to 20 bar in the presence of a catalyst, whose BET surface area is from 20 to 150 m²/g and whose catalytically active mass contains from 80 to 99.9 % by weight of zirconium oxide and from 0.1 to 20 % by weight of one or more lanthanide elements.

2. A process for preparing aromatic aldehydes as claimed in claim 1, wherein the catalyst contains as the catalytically active mass from 90 to 99.9 % by weight of zirconium oxide and as the lanthanide element from 0.1 to 10 % by weight of lanthanum, cerium, praseodymium, neodymium, samarium, europium or mixtures thereof.

3. A process for preparing aromatic aldehydes as claimed in claim 1, wherein the catalyst contains as the catalytically active mass from 92 to 99 % by weight of zirconium oxide and from 1 to 8 % by weight of lanthanum(III) oxide.

4. A process for preparing aromatic aldehydes as claimed in claim 1, wherein the zirconium dioxide is monoclinic.

5. A process for preparing aromatic aldehydes as claimed in claim 1, wherein benzoic acid, 2-methylbenzoic acid, 3-methylbenzoic acid, 4-methylbenzoic acid, 4-isopropylbenzoic acid, 4-methoxybenzoic acid, phthalide or terephthalic acid or their esters are hydrogenated to give the corresponding mono- or dialdehyde.

6. A process for preparing aromatic aldehydes as claimed in claim 1, wherein the molar ratio of hydrogen to carboxylic acid (ester) is from 2:1 to 100:1.

7. A process for preparing aromatic aldehydes as claimed in claim 1, wherein the reaction is carried out in a solid bed.

## Revendications

1. Procédé de préparation d'aldéhydes aromatiques de formule générale I 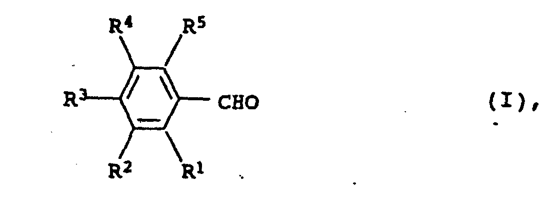 dans laquelle
R¹, R², R³, R⁴ et R⁵ représentent un atome d'hydrogène, un groupement alkyle en C₁-C₆, cycloalkyle en C₃-C₈, aryle, hydroxyle, alcoxy en C₁-C₄, phénoxy, (alkyle en C₇-C₁₂)phényle, (phényle en C₇-C₁₂)alkyle, amino, méthylamino, diméthylamino ou un atome d'halogène et
R², R³ ou R⁴ représentent en outre un groupement formyle ou COOR⁶
R⁶ représente un atome d'hydrogène, un groupement alkyle en C₁-C₆, cycloalkyle en C₃-C₈, aryle, (alkyle en C₇-C₁₂)phényle ou (phényle en C₇-C₁₂)alkyle,
caractérisé en ce que l'on fait réagir des acides carboxyliques aromatiques ou leurs esters de formule générale II 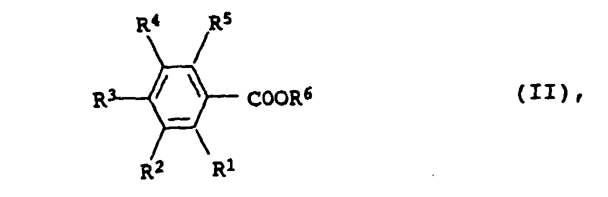 dans laquelle
R¹, R², R³, R⁴, R⁵ et R⁶ prennent la signification susmentionnée ou dans laquelle
R², R³, R⁴ et R⁵ prennent la signification susmentionnée et
R⁶ et R¹ forment ensemble un pont -CH₂-,
avec de l'hydrogène en phase gazeuse à des températures de 200-450°C et sous des pressions de 0,1-20 bar, en présence d'un catalyseur, dont la surface BET s'élève à 20-150 m²/g et dont la masse catalytique active contient 80-99,9% en poids d'oxyde de zirconium et 0,1-20% en poids d'un ou plusieurs éléments de la série des lanthanides.

2. Procédé de préparation d'aldéhydes aromatiques selon la revendication 1, caractérisé en ce que le catalyseur contient, en tant que masse catalytique active, 90-99,9% en poids d'oxyde de zirconium et, en tant qu'élément de la série des lanthanides, 0,1-10% en poids de lanthane, de cérium, de praséodyme, de néodyme, de samarium, d'europium ou de leurs mélanges.

3. Procédé de préparation d'aldéhydes aromatiques selon la revendication 1, caractérisé en ce que le catalyseur contient, en tant que masse catalytique active, 92-99% en poids d'oxyde de zirconium et 1-8% en poids d'oxyde de lanthane III.

4. Procédé de préparation d'aldéhydes aromatiques selon la revendication 1, caractérisé en ce que l'oxyde de zirconium est monoclinique.

5. Procédé de préparation d'aldéhydes aromatiques selon la revendication 1, caractérisé en ce que l'on hydrogène de l'acide benzoïque, de l'acide 2-méthylbenzoïque, de l'acide 3-méthylbenzoïque, de l'acide 4-méthylbenzoïque, de l'acide 4-isopropylbenzoïque, de l'acide 4-méthoxybenzoïque, du phtalide ou de l'acide téréphtalique ou leurs esters pour obtenir les mono- ou dialdéhydes correspondants.

6. Procédé de préparation d'aldéhydes aromatiques selon la revendication 1, caractérisé en ce que le rapport molaire de l'hydrogène à l'acide carboxylique (ester) s'élève de 2 : 1 à 100: 1.

7. Procédé de préparation d'aldéhydes aromatiques selon la revendication 1, caractérisé en ce que l'on mène la réaction dans un lit fixe.
